# EUROPEAN PATENT SPECIFICATION

(11) **EP 3 072 503 B1**
(45) Date of publication and mention of the grant of the patent: **23.09.2020**
(21) Application number: 16161188.4
(22) Date of filing: 18.03.2016
(51) Int. Cl.: A61K 31/685, A61K 31/164, A61K 31/4415, A61K 31/51, A61K 31/714, A61K 9/00, A61K 47/36, A61P 27/02

(54) **OPHTHALMIC COMPOSITION FOR THE CORNEAL PROTECTION**
OPHTHALMISCHE ZUSAMMENSETZUNG FÜR HORNHAUTGEWEBE
COMPOSITION OPHTALMIQUE POUR LA PROTECTION DE LA CORNÉE

(30) Priority: 26.03.2015 IT RM20150128
(43) Date of publication of application: 28.09.2016
(73) Proprietor: D.M.G. Italia Srl, 00071 Pomezia (Rome) (IT)
(72) Inventor: Mercuri, Luigi, 00071 Pomezia (Rome) (IT)
(74) Representative: Hoffmann Eitle

(56) References cited:
- EP-A2- 2 070 518
- WO-A1-03/049747
- WO-A1-2012/000055
- US-A1- 2012 135 087
- HOFFMAN JAY R ET AL: "The effects of acute and prolonged CRAM supplementation on reaction time and subjective measures of focus and alertness in healthy college students", JOURNAL OF THE INTERNATIONAL SOCIETY OF SPORTS NUTRITION, BIOMED CENTRAL LTD, LO, vol. 7, no. 1, 15 December 2010 (2010-12-15), page 39, XP021089480, ISSN: 1550-2783, DOI: 10.1186/1550-2783-7-39
- Review ET AL: "A review of polyvinyl alcohol derivatives: Promising materials for pharmaceutical and biomedical applications", African Journal of Pharmacy and Pharmacology, vol. 8, no. 24, 29 June 2014 (2014-06-29), pages 674-684, XP055409347, NG ISSN: 1996-0816, DOI: 10.5897/AJPP2013.3906

## Description

### Field of the invention

The present invention relates to the pharmaceutical field applied to the protective treatment of the ocular cornea. The present invention finds application in particular in the field of preventive therapy for the preservation, and in the treatment for the recovery, of the good physiological condition and innervations of the corneal epithelium.

### Background of the invention

The corneal epithelium is the outermost layer of the five which totally constitute the cornea, the other layers are Bowman's membrane, stroma, Descemet's membrane and endothelium; it is in contact with the tear film and is essential for the integrity of the cornea.

An alteration of the corneal epithelium (corneal epitheliopathy) may cause pain and the unpleasant feeling of the presence of a foreign body, typically sand, in the eye, increasing with the opening and closing of the eyelids. The pain is due to the discontinuities created in the epithelium, leaving uncovered the nerve terminations which, in turn, are damaged. Alteration of the corneal epithelium may also cause blurred vision. Furthermore, an injury of the cornea integrity makes the cornea and the whole eye more vulnerable to infection. Finally, the damage of the nerve fibers exposes the cornea to neurotrophic lesions that tend to become chronic.

The causes of corneal epitheliopathy are various: acquired corneal dystrophies, corneal inflammation, allergic conjunctivitis, alterations of the tear film, such as Sjogren's syndrome. This latter condition affects the lacrimal glands which reduce tears secretion, and then, the corneal epithelium, no longer optimally protected and lubricated, dries and degenerates. Whenever poor lacrimation and, thus, decreased protection of the epithelium is established, damages and injuries caused, for example, by foreign bodies in the eye, wind, use of not neutral soaps or shampoos, cosmetics, etc., more easily occurs. Other subjects exposed to high risk for corneal epitheliopathy are those wearing contact lenses, moped riders who do not wear protective eyewear, postmenopausal women suffering from dry eye syndrome. Improper use of contact lenses can cause epithelial damage: if too long worn they may cause reduced oxygen supply (hypoxia), creating an epithelial edema. In case of alteration of tearing the contact lens may cause micro-traumas in the transparent outer surface of the eye.

In all these cases therapy can be based on the simple administration of artificial tears, topic antibiotics and ri-epithelizing agents.

Alpha-Glyceryl Phosphoryl Choline (Alpha-GPC), also known as choline alfoscerate, has long been used in therapy as a precursor of acetylcholine and phosphatidylcholine. The rationale of the use thereof is duplex: on one hand it increases the brain acetylcholine content and the related memory processes, on the other hand it promotes the derivate synthesis of phosphatidylcholine and sphingolipids, improving the trophism of neuronal membranes and myelin sheaths. Alpha-GPC is therefore used as a supplement to treat cognitive disorders linked to the onset of Alzheimer's disease, stroke, heart attack or dementia. However, it is believed that Alpha-GPC can naturally stimulate the secretion of the growth hormone and of the trophic IGF-1 factor, resulting in increased muscle mass and performance, improved recovery capacity and delay of sarcopenia due to aging.

The International patent application WO 2012/073191 (US 2012/0135087) discloses the use of topical compositions containing the Alpha-GPC to maintain and restore the integrity of various types of mucous membranes, including the ocular mucosa, in some embodiments it also involves the presence of sodium hyaluronate. This invention is based on the concept of "rheological synergy" between GPC and hyaluronate, which, when mixed together, result in formulations with viscosities as to increase the permanence of the individual compounds in the eye and then their therapeutic properties.

The invention disclosed in the International patent application WO 2003/049747 relates to a pharmaceutical composition that contains at least panthenol, and/or pantothenic acid, hyaluronic acid, and/or hyaluronate, and optionally pharmaceutical adjuvants. The invention further relates to the use of the pharmaceutical composition for treating ophthalmologic and/or rhinologic dysfunctions.

However, in the relative technology field, it is still very felt the need of original ocular compositions compared to those of the state of the art for the protection of the corneal epithelium.

In this respect, it is interesting to note that according to the prior art teachings it is possible increasing the synthesis of corneal phosphatidylcholine by administering its exogenous metabolic precursors, such as GPC and citicoline (CTP-choline). The skilled in the field, willing to increase the synthesis of phosphatidylcholine by topical corneal therapies enabling more efficient and alternatives metabolic pathways, cannot find proper disclosures. The problem to be solved is therefore to identify innovative corneal formulations, able to act topically on not yet explored metabolic pathways, and potentially able of improving the corneal trophism.

### Description of the figures

Figure 1 is the diagram showing the variation of the number of the nervous fibers as revealed in IVCM images from the two tested groups.
Figure 2 is the diagram showing the variation of the number of bead-shape formation in 1mm of nervous fiber as revealed in IVCM images from the two tested groups.
Figure 3 is showing the morphological analysis of the corneal epithelium by in vivo confocal microscopy (IVCM), particularly showing the presence of alterations of the superficial epithelium with cells having over reflective nucleus and a moderate stromal edema with over reflective activated keratocytes in the incision area.
Figure 4 is showing the morphological analysis of the corneal epithelium by in vivo confocal microscopy (IVCM), particularly showing the typical innervations of the tissue.

### Detailed description of the invention

The object of the present invention is to provide an ophthalmic composition according to claim 1, comprising the association of Alpha-Glyceryl Phosphoryl Choline, vitamin and provitamin factors of group B, and at least one modifying agent of the rheological properties of the composition, and said ophthalmic composition for use in the treatment of the corneal epithelium, such as acquired corneal dystrophies, corneal inflammations, allergic conjunctivitis, alterations of the tear film, reduction of tear secretion, micro-traumas, traumas from contact lenses, post refractive surgery trauma, corneal ulcers.

The vitamin and provitamin factors of the composition according to the invention are those of the B group, and are vitamin B6, vitamin B12 and D-panthenol. The D-panthenol present in the formulation favors the production of triglycerides necessary for the synthesis of phospholipids, while the addition of vitamin B6 prevents the accumulation of homocysteine, cytotoxic metabolic derivative, enabling its transformation into the protective compound glutathione.

According to the invention the rheological agent present in the composition is: a cellulose derivative, carboxymethyl cellulose (CMC), hydroxypropylethyilcellulose, hydroxyethylcellulose, polyvinyl alcohol (PVA) and its derivatives, xanthan gum, a thermogelling polymer-s, the EG56 copolymer (copolymer Bis-Methoxy PEG-13, PEG-438/PPG-110 SMDI), hyaluronic acid or a salt thereof.

In a particularly preferred embodiment the rheological agent is hyaluronic acid, or a salt thereof. The high viscosity and the high capacity of binding water of hyaluronic acid in the association of the composition is able to protect and lubricate the ocular surface.

The composition according to the invention has a viscosity ranging between 6.2 and 8.2 cSt, preferably the composition has a viscosity of 7.2 cSt.

Therefore, the composition according to the invention, by the synergy of its components, provides a specific ophthalmic product able to protect the cornea and exerts a particularly significant neurotrophic effect. It is indicated in case a neurotrophic action at the level of the corneal tissue is necessary, even in the presence of traumatic events. Such neurotrophic effect joins and enhances the action of vitamins and pro vitamins B that favor the replication and reconstruction process of the epithelial cells. These effects synergistically supplements the alpha-glyceryl phosphoryl choline action, which, as a precursor and natural metabolite of phospholipids, facilitates the formation of new cells and the skin restoring function, restoring the chemical structure of cell membranes.

The use of the composition according to the invention enables to restore the natural balance of the corneal epithelium, to correct and eliminate the effects of alteration of tissue that have to protect the cornea from external agents, and helps to prevent the eye discomfort due to various causes.

The protective properties of the composition according to the invention are due to the synergic effect of hyaluronic acid, alpha-glyceryl phosphoryl choline, vitamin B6, B12 and D-panthenol.

One hundred gram of the ophthalmic composition according to the invention comprise hyaluronic acid, or a salt thereof, in a variable amount between 0.01 and 1 g, preferably 100 g of composition comprise 0.15 g of hyaluronic acid, or a salt thereof.

The amount of D-panthenol present in 100 g of ophthalmic composition varies between 0.1 and 2.0 g, preferably 100 g of the composition comprise an amount of D-panthenol equal to 1.0 g.

The amount of alpha-glyceryl phosphoryl choline present in 100 g of ophthalmic composition varies between 0.01 and 1.0 g, preferably 100 g of composition comprise an amount of alpha-glyceryl phosphoryl choline equal to 0.2 g.

One hundred gram of the ophthalmic composition according to the invention comprise vitamin B6 and vitamin B12, each in variable amount from 0.001 to 0.01 g, preferably vitamin B6 and vitamin B12 are present in 100 g of composition in an amount of 0.002 g each.

The active ingredients constituting the mixture used in the present invention, in particular hyaluronic acid, or its salts, and alpha-glyceryl phosphoryl choline, are already known for their individual viscosing, lubricant, and skin restoring abilities, however the innovative teaching according to the invention is that the combination of such active principles, and in particular the contribution of the B group vitamins, provides the final product with a superior efficacy than expected. This has been experimentally demonstrated, in particular with respect to subjects for which the treatments carried out with the individual active principles have proved to be unsatisfactory.

In addition to the association of hyaluronic acid, alpha-glyceryl phosphoryl choline, vitamin B6, B12 and panthenol the ophthalmic composition for the protection of the cornea according to the invention further comprises, according to an embodiment of such ophthalmic composition, at least one or more pharmaceutically accepted excipients, solvents, preservatives, acidity regulators, chelating agents, osmotic agents and pH stabilizers.

According to the invention any buffering system known to the person skilled useful to stabilize the pH value of the final composition can be used, preferably the system sodium phosphate monobasic/dibasic is used.

The ophthalmic composition according to the invention has a pH value between 7.0 and 7.5, preferably it is an isotonic buffered composition having pH 7.20.

The ophthalmic composition according to the invention has a density of between 1.000 and 1.05 g/ml, preferably has a density of 1.015 g/ml.

The osmolality of the composition according to the invention is ranging between 250 and 350 mOsm/Kg, preferably the composition has osmolality of 300 mOsm/Kg. The composition according to the invention is in a form suitable to be applied topically in the eye, in liquid form having various levels of viscosity, such as solution, drops, gel, ointment, cream, paste.

The invention will now be illustrated with reference to non-limiting formulation examples and experimental assays described below.

### Example 1 - Ophthalmic Solution

In a particularly preferred embodiment of the invention 100 g of composition has the following formulation:

| Ingredient: | Amount (g) |
|---|---|
| Hyaluronic acid sodium salt | 0.15 |
| D-panthenol | 1.00 |
| Vitamin B6 (pyridoxine hydrochloride) | 0.002 |
| Vitamin B12 (cyanocobalamin) | 0.002 |
| Alpha-glyceryl phosphoryl choline | 0.2 |
| Microglicin-50 | 0.004 |
| EDTA disodium salt | 0.10 |
| Potassium hydroxide | 0.006 |
| Sodium phosphate monobasic | 0.15 |
| Sodium phosphate dibasic | 0.40 |
| Sodium chloride | 0.48 |
| Distilled water | 97.506 |

### Example 2 - Stability

The stability of the composition according to the invention having formulation as set forth in Example 1 was evaluated in experimental accelerated aging conditions for a period of six months, at 30-day intervals, observing physical-chemical and microbiological parameters indicated in the table below.

| **Time** | **Appearence** | **pH** | **Viscosity at 25°C** (cSt) | **Density at 20°C** (g/ml) | **Osmolality** (mOsm/Kg) |
|---|---|---|---|---|---|
| T₀ | clear viscous pink and odorless solution | 7,20 | 7,68 | 1,010 | 302 |
| T₃₀ | clear viscous pink and odorless solution | 7,18 | 7,55 | 1,011 | 307 |
| T₆₀ | clear viscous pink and odorless solution | 7,23 | 7,10 | 1,011 | 311 |
| T₉₀ | clear viscous pink and odorless solution | 7,17 | 7,06 | 1,010 | 317 |
| T₁₂₀ | clear viscous pink and odorless solution | 7,17 | 6,40 | 1,010 | 322 |
| T₁₈₀ | clear viscous pink and odorless solution | 7, 16 | 6,76 | 1,010 | 328 |

### Example 3 - In vivo confocal microscopy corneal of the human cornea after administration of the composition in patients undergoing cataract surgery._

### Rational

Cataract surgery may results in ocular discomfort determined by a multiplicity of factors such as surgical trauma, pre-existing local therapies and misrecognized problems of the ocular surface. The surgical incisions are certainly one of the causes of ocular surface alterations related to discontinuities of the corneal nervous component and epithelial alteration (1). *In vivo* confocal microscopy (IVCM) is a diagnostic procedure allowing to evaluate disordes of the cornea and conjunctiva as it provides details of the ocular structures at the cellular level (2). It represents an useful investigation technique to evaluate the processes that accompany postoperative changes occurring in the corneal incisions.

The purpose of the study is to describe by IVCM the effects of the topical composition comprising alpha-glyceryl phosphoryl choline and D-panthenol as in Example 1 on the morphology of the corneal tissue adjacent to the incision in patients undergoing cataract surgery.

### Methods

A prospective, randomized, blind study was conducted on 40 eyes of 40 patients undergoing standard phacoemulsification with intraocular implantation of foldable lens (IOL) in the capsular bag at Department of Surgical Ophthalmology of University of Siena. All the enrolled patients gave their consent to the study.

All patients received as prophylaxis treatment for endophthalmitis a third generation quinolone topical antibiotic starting the treatment three days before surgery and for one week after surgery; for prophylaxis of cystoid macular edema all patients received a NSAID eye drops starting 3 days before surgery and for 20 days after surgery. In addition a topical association antibiotic-steroid was used for 20 days after surgery.

In order to carry out the study, patients who underwent phacoemulsification with IOL implantation in the capsular bag by injector were enrolled.

From the study patients who received interventions with incisions longer than 2.2 mm or intraoperative complications, patients with corneal dystrophy, with ocular surface diseases, with clinical history of uveitis, diabetes, postoperative inflammation, glaucoma or ocular hypertension higher than 21 mmHg, patients taking topical antiglaucomatous therapy, and using contact lenses were excluded; patients who at the end of intervention received a suture at the incision site were excluded as well.

The enrolled patients were divided into 2 groups of 20 patients each.

Group 1 received the topical treatment with the composition comprising alpha-glyceryl phosphoryl choline and D-panthenol having formulation according to Example 1, 3 times a day starting from the first day after surgery and continued the treatment for 1 month;

Group 2 received the postoperative topical therapy based on the administration of a hyaluronate sodium collirium 3 times a day for 1 month.

Each patient was evaluated the day before the surgical IOL implantation (baseline), the day following the implantation, and the 30th, 60th and 90th postoperative day, the patient clinical examination was performed by slit lamp, Schirmer test, break-up time (BUT), and IVCM (HRTII with Rodstock cornea module, Heidelberg Eng., Germany). Hence, all patients eyes were analyzed by laser scanning confocal microscope (HRT II Rostock Corneal Module, Heidelberg Engineering, Germany), using a laser diode source at 670-nm wavelength. The resulting digital images measure 400µm x 400 µm.

All IVCM testings were performed by the same operator in order to examine the peripheral cornea in the pre- and post-operative time. The examination was carried out under topical anesthesia using oxybuprocaine and a gel eye drop (Recugel, B&L) instilled between the polymethyl methacrylate capsule and the objective of the confocal microscope in the lower conjunctival fornix. During the examination all patients were invited to set a nasal fixation point to better visualize the incision and minimize the optical distortions in the incision area. For each examined eye at least 20 images of corneal epithelium, the nerve plexus sub-basal and stromal were obtained. The three most representative images were evaluated. The following parameters were considered:
- Density of the nerves of the temporal nerve fibers, defined as the sum of the nerve fibers present in an image.
- Number of bead-shape formations along 1 mm of nerve fiber.

A qualitative analysis of the morphological alterations in the epithelial, stromal and endothelial layer in the incision area was also performed. All surgical interventions were performed by the same surgeon: under topical anesthesia a 2.2 mm sutureless temporal clear corneal tunnel was created; particular care was dedicated to the incision. A standard phacoemulsification with IOL implantation (SN60WF) in the capsular bag using the injection system monarch II with a C format cartridge (Alcon, Texas, USA) was performed. After the surgery a stromal hydration of the main and lateral incisions with an assessment of the absence of leakage was performed. The results obtained by the two groups presented as mean and standard deviation were subjected to statistical analysis through the use of the t test for unpaired data with p < 0.05.

### Results

After the surgery, in both groups a worsening of the ocular surface stability, as evidenced by BUT and Schirmer test was observed. In both groups during follow-up period all the parameters have progressively improved, but the use of the composition according to the invention comprising alpha-glyceryl phosphoryl choline and D-panthenol was associated with a faster recovery from the intervention characterized by faster improvement of the signs and symptoms of the alteration of the ocular surface.

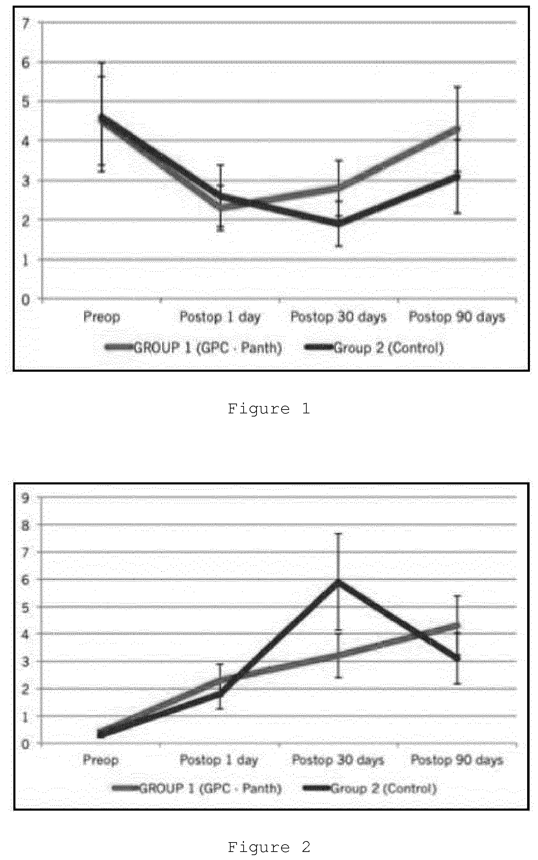

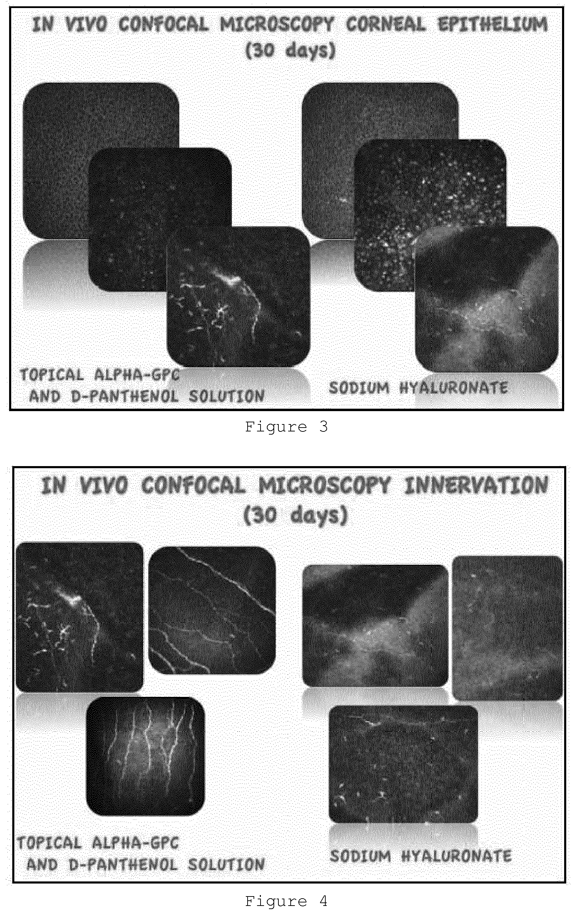

Figure 1 shows that following cataract surgery, especially in the first month after surgery; a significant reduction of the density of nerve fibers occurs. Compared to the preoperative values in both groups a reduced nerve fiber density is evident. However, the use of the composition is associated with a higher density of nerve fibers at 1 and 3 months compared to the control group (Fig. 1). In the control group (Group 2) the number of bead shape formations is higher one month postoperatively (Fig. 2). Thirty days after surgery, the morphological analysis of the corneal incision has highlighted the presence of alterations of the superficial epithelium showing cells having over reflective nucleus and a moderate, but reduced, stromal edema still with over reflective activated keratocytes in the incision area especially in group 1 (Fig. 3). Endothelial cells with normal morphology are in the incision site. The sub-basal nerve plexus presents winding fibers, over reflective and reduced in number (Fig. 4).

### Discussion

In recent years, the IVCM has been more and more used for studying the morphological changes of the ocular surface, as it allows *in vivo* microstructural and non-invasive analysis of the human cornea and consequently its alterations (4-5). The cornea is a densely innervated tissue receiving sensory and autonomic (sympathetic and parasympathetic) nerve fibers. The surgical procedures of the anterior segment induce iatrogenic damage to the corneal nerve structures associated with alterations of the ocular surface (3). Although the cataract surgical procedure is well standardized the operator expertise is an important factor in the construction of engraving. In our study, all the incisions were performed by the same surgeon in the temporal part of the eye.

The purpose of this study is to compare by IVCM the clinical and morphological outcomes in patients underwent cataract surgery treated by topical administration of the composition according to the invention and in a control group of patients treated with sodium hyaluronate. The study has demonstrated the efficacy of the composition at corneal incision site, also providing a systematic and objective evaluation of the cornea based on parameters that can be analyzed quantitatively and qualitatively by images. The cataract surgery at the incision site involves a degeneration of the nerve fibers with consequent reduction of the nerve density (6). A recent study showed a significant reduction of the nerve fibers one month after surgery and a return to preoperative values 8 months after surgery (7). It has been shown that the surgery halves the density of the temporal nerve fibers and reduces by 25-35% the central ones. The study has shown that in cataract surgery the corneal incisions cause alterations in the nerve fiber layer and thus may be responsible for the symptoms that often accompanies the post-operative course. Furthermore, it has shown that the use of a topical composition comprising alpha-glyceryl phosphoryl choline and D-panthenol accelerates postoperative recovery. Changes in the density of nerve fibers highlighted by IVCM meet clinical results obtained. By the topical administration of the composition according to the invention symptoms can reduced by improving corneal tropism and accelerating the repair mechanisms.

The IVCM data are correlated and confirmed by clinical signs and symptoms. Immediately after surgery the central corneal sensitivity is reduced by approximately 10% and the ocular surface worsens as demonstrated by Schirmer test and BUT (8). In our study, the clinical results, including the BUT test, has shown statistically significant differences between the group treated with the composition according to the invention and the control group.

A large number of bead-shape formation in the nerve fibers may instead be related to the high metabolic activity in repairing the epithelial damage. For example, the large number of these formations observed in the dry eye could be an attempt to increase and compensate and the altered epithelial tropism (5).

Clinical data and morphology as highlighted in our study by IVCM show a better pattern in the group treated with the composition comprising alpha-glyceryl phosphoryl choline and D-panthenol compared to the untreated group. In conclusion, topical treatment based on the composition according to the invention has proved to be well tolerated, favoring and stabilizing the re-epithelialization process and able of accelerating and modulating the corneal innervation repair.

### References

1. Paton D., Ryan S. Present Trends in Incision and Closure of the Cataract Wound. Miami: Highlights of Ophthalmology; 1973: 3-10.
2. Cavanagh H.D., et al. Clinical and diagnostic use of in vivo confocal microscopy in patients with corneal disease. Ophthalmology 1993; 100: 1444-54.
3. Oliveira-Soto L., Efron N. Morphology of corneal nerves using confocal microscopy. Cornea. 2001; 20: 374-384.
4. Grupcheva C. N., et al. Assessing the sub-basal nerve plexus of the living healthy human cornea by in vivo confocal microscopy. Clin Exp Ophthalmol. 2002; 30: 187-190.
5. Benitez del Castillo J. M., et al. An in vivo confocal masked study on corneal epithelium and subbasal nerves in patients with dry eye. Invest Ophthalmol Vis Sci. 2004; 45: 3030-3035.
6. Kohnen T., Koch D.D. Experimental and clinical evaluation of incision size and shape following forceps and injector implantation of a three-piece high refractive index silicone intraocular lens. Graefes Arch Clin Exp Ophthalmol. 1998; 236: 922-928.
7. DeCIlla S., et al. Corneal involvement in uneventdul cataract surgery: an in vivo confocal microscopy study. Ophthalmologica 2014; 231:103-10.
8. Kelman C.D. Preface. In: Alio JL, Rodriguez Prats JL, Galal A, eds. MICS Micro-incision Cataract Surgery. Miami: Highlights of Ophthalmology; 2004.

## Claims

1. An Ophthalmic composition comprising an association of Alpha-Glyceryl-Phosphoryl-Choline, vitamin and pro-vitamin factors belonging to group B and a rheological agent commonly used in ophthalmic compositions; wherein vitamin and pro-vitamin factors belonging to group B are vitamin B6, vitamin B12 and D-panthenol;and
wherein the rheological agent is: a cellulose derivative, carboxymethyl cellulose (CMC), hydroxypropylethyl cellulose, hydroxyethyl cellulose, polyvinyl alcohol (PVA) and its derivatives, xanthan gum, a thermogelling polymer, Bis-Methoxy PEG-13, PEG-438/SMDI PPG-110 copolymer, hyaluronic acid or a salt thereof.

2. Ophthalmic composition according to claim 1 comprising an association of Alpha-Glyceryl-Phosphoryl-Choline, vitamin B6, vitamin B12, D-panthenol and hyaluronic acid or a salt thereof.

3. Ophthalmic composition according to claim 1 wherein in 100 g of composition the amount of rheological agent varies between 0.01 and 1 g, the amount of D-panthenol varies between 0.1 and 2.0 g, the alpha glyceryl phosphoryl choline is present in an amount ranging between 0.01 and 1.0 g, and the amount of vitamin B6 and vitamin B12 ranges between 0.001 and 0.01 g.

4. Ophthalmic composition according to claim 3 in which in 100 g of the composition the amount of rheological agent is 0.15 g, the amount of D-panthenol is 1.0 g, the alpha glyceryl phosphoryl choline is present in an amount equal to 0.2 g, and each vitamin is present in an amount of 0.002 g.

5. Ophthalmic composition according to claims 1 to 4, **characterized in that** it further comprises solvents, preservatives, acidity regulators, chelating agents, osmotic agents and pH stabilizers.

6. Ophthalmic composition according to claim 1 to 5, **characterized in that** it has pH value ranging between 7.0 and 7.5, density ranging between 1.0 and 1.05 g/ml, osmolality ranging between 250 and 350 mOsm/Kg.

7. Ophthalmic composition according to claims 1 to 6, **characterized in that** it has the following formulation:
| | |
|---|---|
| Hyaluronic acid sodium salt | 0.15 g |
| D-panthenol | 1.00 g |
| Vitamin B6 (pyridoxine hydrochloride) | 0.002 g |
| Vitamin B12 (cyanocobalamin) | 0.002 g |
| Alpha-glyceryl phosphoryl choline | 0.2 g |
| Microglicin-50 | 0.004 g |
| EDTA disodium salt | 0.10 g |
| Potassium hydroxide | 0.006 g |
| Monobasic sodium phosphate | 0.15 g |
| Dibasic sodium phosphate | 0.40 g |
| Sodium chloride | 0.48 g |
| Distilled water | 97.506 g |

8. Ophthalmic composition according to claims 1 to 7, **characterized in that** it is in liquid form suitable for topical administration in the eye, with a viscosity ranging between 6.2 and 8.2 cSt.

9. Ophthalmic composition according to claims 1 to 8 for use in the treatment of corneal epithelium disorders.

10. Ophthalmic composition for use according to claim 9 wherein the corneal epithelium disorders are: acquired corneal dystrophies, corneal inflammation, allergic conjunctivitis, alterations of the tear film, reduction of tear secretion, micro-traumas, traumas from contact lenses, post refractive surgery trauma, corneal ulcers.

## Patentansprüche

1. Ophthalmische Zusammensetzung, umfassend eine Kombination aus Alpha-Glycerylphosphorylcholin, Vitamin- und Provitaminfaktoren, die zur B-Gruppe gehören, und ein rheologisches Mittel, das üblicherweise in ophthalmischen Zusammensetzungen verwendet wird; worin Vitamin- und Provitaminfaktoren, die zur B-Gruppe gehören, Vitamin B6, Vitamin B12 und D-Panthenol sind; und worin das rheologische Mittel
ein Cellulosederivat, Carboxymethylcellulose (CMC), Hydroxypropylethylcellulose, Hydroxyethylcellulose, Polyvinylalkohol (PVA) und dessen Derivate, Xanthangummi, ein thermogelierendes Polymer, Bis-Methoxy PEG-13, PEG-438/SMDI PPG-110 Copolymer, Hyaluronsäure oder ein Salz davon ist.

2. Ophthalmische Zusammensetzung gemäß Anspruch 1, umfassend eine Kombination aus Alpha-Glycerylphosphorylcholin, Vitamin B6, Vitamin B12, D-Panthenol und Hyaluronsäure oder ein Salz davon.

3. Ophthalmische Zusammensetzung gemäß Anspruch 1, worin in 100 g der Zusammensetzung die Menge des rheologischen Mittels zwischen 0,01 und 1 g variiert, die Menge des D-Panthenols zwischen 0,1 und 2,0 g variiert, das Alpha-Glycerylphosphorylcholin in einer Menge im Bereich zwischen 0,01 und 1,0 g vorhanden ist und die Menge des Vitamin B6 und des Vitamin B12 im Bereich zwischen 0,001 und 0,01 g liegt.

4. Ophthalmische Zusammensetzung gemäß Anspruch 3, in der in 100 g der Zusammensetzung die Menge des rheologischen Mittels 0,15 g beträgt, die Menge des D-Panthenols 1,0 g beträgt, das Alpha-Glycerylphosphorylcholin in einer Menge von 0,2 g vorhanden ist und jedes Vitamin in einer Menge von 0,002 g vorhanden ist.

5. Ophthalmische Zusammensetzung gemäß den Ansprüchen 1 bis 4, **dadurch gekennzeichnet, dass** sie ferner Lösungsmittel, Konservierungsmittel, Säureregulatoren, Chelatbildner, osmotische Mittel und pH-Stabilisatoren umfasst.

6. Ophthalmische Zusammensetzung gemäß den Ansprüchen 1 bis 5, **dadurch gekennzeichnet, dass** sie einen pH-Wert im Bereich zwischen 7,0 bis 7,5, eine Dichte im Bereich zwischen 1,0 und 1,05 g/ml und eine Osmolalität im Bereich zwischen 250 bis 350 mOsm/kg aufweist.

7. Ophthalmische Zusammensetzung gemäß den Ansprüchen 1 bis 6, **dadurch gekennzeichnet, dass** sie die folgende Formulierung aufweist:
| | |
|---|---|
| Hyaluronsäure Natriumsalz | 0,15 g |
| D-Panthenol | 1,00 g |
| Vitamin B6 (Pyridoxinhydrochlorid) | 0,002 g |
| Vitamin B12 (Cyanocobalamin) | 0,002 g |
| Alpha-Glycerylphosphorylcholin | 0,2 g |
| Microglycin 50 | 0,004 g |
| EDTA Dinatriumsalz | 0,10 g |
| Kaliumhydroxid | 0,006 g |
| Monobasisches Natriumphosphat | 0,15 g |
| Dibasisches Natriumphosphat | 0,40 g |
| Natriumchlorid | 0,48 g |
| Destilliertes Wasser | 97,506 g |

8. Ophthalmische Zusammensetzung gemäß den Ansprüchen 1 bis 7, **dadurch gekennzeichnet, dass** sie in flüssiger Form vorliegt, die für die topische Verabreichung am Auge geeignet ist, mit einer Viskosität im Bereich zwischen 6,2 bis 8,2 cSt.

9. Ophthalmische Zusammensetzung gemäß den Ansprüchen 1 bis 8 zur Verwendung bei der Behandlung von Erkrankungen des Hornhautepithels.

10. Ophthalmische Zusammensetzung zur Verwendung gemäß Anspruch 9, worin die Erkrankungen des Hornhautepithels erworbene Hornhautdystrophien, Hornhautentzündung, allergische Bindehautentzündung, Veränderungen des Tränenfilms, Verminderung der Tränensekretion, Mikrotraumata, Traumata durch Kontaktlinsen, Traumata nach refraktiver Chirurgie, Hornhautgeschwüre sind.

## Revendications

1. Composition ophtalmique comprenant une association d'alpha-glycéryl-phosphoryl-choline, de facteurs vitaminiques et pro-vitaminiques appartenant au groupe B et d'un agent rhéologique communément utilisé dans les compositions ophtalmiques ; dans laquelle les facteurs vitaminiques et pro-vitaminiques appartenant au groupe B sont la vitamine B6, la vitamine B12 et le D-panthénol ; et
dans laquelle l'agent rhéologique est : un dérivé de cellulose, la carboxyméthylcellulose (CMC), l'hydroxypropyléthylcellulose, l'hydroxyéthylcellulose, le poly(alcool vinylique) (PVA) et ses dérivés, la gomme xanthane, un polymère thermogélifiant, le bis-méthoxy PEG-13, un copolymère de PEG-438/SMDI PPG-110, l'acide hyaluronique ou un sel de celui-ci.

2. Composition ophtalmique selon la revendication 1 comprenant une association d'alpha-glycéryl-phosphoryl-choline, de vitamine B6, de vitamine B12, de D-panthénol et d'acide hyaluronique ou d'un sel de celui-ci.

3. Composition ophtalmique selon la revendication 1, dans laquelle, dans 100 g de composition, la quantité de l'agent rhéologique varie entre 0,01 et 1 g, la quantité du D-panthénol varie entre 0,1 et 2,0 g, l'alpha-glycéryl-phosphoryl-choline est présente en une quantité comprise entre 0,01 et 1,0 g, et la quantité de vitamine B6 et de vitamine B12 est comprise entre 0,001 et 0,01 g.

4. Composition ophtalmique selon la revendication 3, dans laquelle, dans 100 g de composition, la quantité de l'agent rhéologique est de 0,15 g, la quantité du D-panthénol est de 1,0 g, l'alpha-glycéryl-phosphoryl-choline est présente en une quantité égale à 0,2 g, et chaque vitamine est présente en une quantité de 0,002 g.

5. Composition ophtalmique selon les revendications 1 à 4, **caractérisée en ce qu'**elle comprend en outre des solvants, des conservateurs, des régulateurs d'acidité, des agents chélatants, des agents osmotiques et des agents stabilisant le pH.

6. Composition ophtalmique selon les revendications 1 à 5, **caractérisée en ce qu'**elle a une valeur de pH comprise entre 7,0 et 7,5, une densité comprise entre 1,0 et 1,05 g/ml, une osmolalité comprise entre 250 et 350 mOsm/kg.

7. Composition ophtalmique selon les revendications 1 à 6, **caractérisée en ce qu'**elle a la formulation suivante :
| | |
|---|---|
| sel sodique d'acide hyaluronique | 0,15 g |
| D-panthénol | 1,00 g |
| vitamine B6 (chlorhydrate de pyridoxine) | 0,002 g |
| vitamine B12 (cyanocobalamine) | 0,002 g |
| alpha-glycéryl-phosphoryl-choline | 0,2 g |
| microglycine-50 | 0,004 g |
| sel disodique d'EDTA | 0,10 g |
| hydroxyde de potassium | 0,006 g |
| phosphate de sodium monobasique | 0,15 g |
| phosphate de sodium dibasique | 0,40 g |
| chlorure de sodium | 0,48 g |
| eau distillée | 97,506 g |

8. Composition ophtalmique selon les revendications 1 à 7, **caractérisée en ce qu'**elle est sous une forme liquide convenant pour une administration topique dans l'œil, avec une viscosité comprise entre 6,2 et 8,2 cSt.

9. Composition ophtalmique selon les revendications 1 à 8, pour une utilisation dans le traitement de troubles de l'épithélium cornéen.

10. Composition ophtalmique pour une utilisation selon la revendication 9, dans laquelle les troubles de l'épithélium cornéen sont : des dystrophies cornéennes acquises, une inflammation cornéenne, une conjonctivite allergique, des altérations du film lacrymal, une réduction de la sécrétion de larmes, des microtraumatismes, des traumatismes dus à des lentilles de contact, un traumatisme post-chirurgie réfractive, des ulcères cornéens.
